# EUROPÄISCHE PATENTSCHRIFT

(11) **EP 3 873 428 B1**
(45) Veröffentlichungstag und Bekanntmachung des Hinweises auf die Patenterteilung: **26.03.2025**
(21) Anmeldenummer: 19798227.5
(22) Anmeldetag: 31.10.2019
(51) Int. Cl.: A61Q 5/12, A61Q 5/00, A61K 8/58, A61Q 5/06, A61K 8/81

(54) **WIRKSTOFFZUSAMMENSETZUNG ZUM SCHUTZ KÜNSTLICH GEFÄRBTER HAARE**
COMPOSITION FOR PROTECTING DYED HAIR
COMPOSITION POUR LA PROTECTION DES CHEVEUX TEINTS

(30) Priorität: 31.10.2018 DE 102018127300
(43) Veröffentlichungstag der Anmeldung: 08.09.2021
(73) Patentinhaber: Henkel AG & Co. KGaA, 40589 Düsseldorf (DE)
(72) Erfinder: KROHN, Rene, 22848 Norderstedt (DE); SCHULZE ZUR WIESCHE, Erik, 33619 Bielefeld (DE)
(86) Internationale Anmeldenummer: PCT/EP2019/079786
(87) Internationale Veröffentlichungsnummer: WO 2020/089371

(56) Entgegenhaltungen:
- EP-A2- 1 767 187
- EP-A2- 2 347 795
- WO-A1-2016/091456
- WO-A1-2017/102856
- DATABASE GNPD [online] MINTEL; 26 November 2008 (2008-11-26), ANONYMOUS: "Anti-Dryness Cleansing Cream", XP055657045, retrieved from www.gnpd.com Database accession no. 1009536
- JOHN WOODRUFF: "Hair care - Crowning glory", 22 November 2012 (2012-11-22), XP055656728, Retrieved from the Internet <URL:https://www.manufacturingchemist.com/technical/article_page/Hair_care__Crowning_glory/82206> [retrieved on 20200110]

## Beschreibung

Die vorliegende Erfindung betrifft kosmetische Mittel zur Behandlung eines keratinischen Materials, wobei das Mittel eine organische Siliciumverbindung und mindestens ein Polyacrylat umfasst, sowie die Verwendung des kosmetischen Mittels.

Die kosmetische Behandlung von Haut und Haaren ist ein wichtiger Bestandteil der menschlichen Körperpflege. So wird menschliches Haar heute in vielfältiger Weise mit haarkosmetischen Zubereitungen behandelt. Dazu gehören etwa die Reinigung der Haare mit Shampoos, die Pflege und Regeneration mit Spülungen und Kuren sowie das Bleichen, Färben und Verformen der Haare mit Färbemitteln, Tönungsmitteln, Wellmitteln und Stylingpräparaten. Dabei spielen Mittel zur Veränderung oder Nuancierung der Farbe des Kopfhaares eine besondere Rolle. Sieht man von den Blondiermitteln, die eine oxidative Aufhellung der Haare durch Abbau der natürlichen Haarfarbstoffe bewirken, ab, so sind im Bereich der Haarfärbung im wesentlichen drei Typen von Haarfärbemitteln von Bedeutung: Für dauerhafte, intensive Färbungen mit entsprechenden Echtheitseigenschaften werden sogenannte Oxidationsfärbemittel verwendet. Solche Färbemittel enthalten üblicherweise

Oxidationsfarbstoffvorprodukte, sogenannte Entwicklerkomponenten und Kupplerkomponenten.

Weisen die im Verlauf der Farbausbildung gebildeten bzw. direkt eingesetzten Farbstoffe deutlich unterschiedliche Echtheiten (z. B. UV-Stabilität, Schweißechtheit, Waschechtheit etc.) auf, so kann es mit der Zeit zu einer erkennbaren und daher unerwünschten Farbverschiebung kommen. Auch häufiges Waschen oder andere Pflegebehandlungen können zu Farbverschiebungen bewirken. Dieses Phänomen tritt verstärkt auf, wenn die Frisur Haare oder Haarzonen unterschiedlichen Schädigungsgrades aufweist. Ein Beispiel dafür sind lange Haare, bei denen die lange Zeit allen möglichen Umwelteinflüssen ausgesetzten Haarspitzen in der Regel deutlich stärker geschädigt sind als die relativ frisch nachgewachsenen Haarzonen.

Für temporäre Färbungen werden üblicherweise Färbe- oder Tönungsmittel verwendet, die als färbende Komponente sogenannte Direktzieher enthalten. Hierbei handelt es sich um Farbstoffmoleküle, die direkt auf das Haar aufziehen und keinen oxidativen Prozess zur Ausbildung der Farbe benötigen. Diese Färbungen sind gegen Shampoonieren in der Regel deutlich empfindlicher als die oxidativen Färbungen, so dass dann sehr viel schneller eine vielfach unerwünschte Nuancenverschiebung oder gar eine sichtbare "Entfärbung" eintritt.

Bei einem weiteren gebräuchlichen Färbeverfahren werden Vorstufen des natürlichen Haarfarbstoffes Melanin auf das Haar aufgebracht; diese bilden dann im Rahmen oxidativer Prozesse im Haar naturanaloge Farbstoffe aus. In solchen Verfahren wird beispielsweise 5,6-Dihydroxyindolin als Farbstoffvorprodukt eingesetzt. Bei, insbesondere mehrfacher, Anwendung von Mitteln mit 5,6-Dihydroxyindolin ist es möglich, Menschen mit ergrauten Haaren die natürliche Haarfarbe wiederzugeben. Die Ausfärbung kann dabei mit Luftsauerstoff als einzigem Oxidationsmittel erfolgen, so dass auf keine weiteren Oxidationsmittel zurückgegriffen werden muss.

Bei allen Haarfärbung treten mehrere Probleme auf. Zum einen kann eine unerwünschte Anfärbung von Hautpartien beim Ausspülen des Haarfärbemittels auftreten. Dieser Effekt kann auch dazu führen, dass hellere "Strähnchen" durch die Haarfarbe beim Auswaschen nachgefärbt werden, oder das Färbemittel beim Auswaschen aus dunkleren "Strähnchen" das hellere Basishaar anfärbt. Das Auswaschen von Farbstoffen ist generell mit dem Nachteil verbunden, dass die ursprünglich gewünschte Farbe sich verändert. Besonders Haarfarben mit Rot- oder Blautönen besitzen eine verkürzte Verweilzeit im Haar, so dass deren Auswaschung zu unerwünschten Farben führen. Diesem Problem wird auch durch Farbschutzprodukte entgegengewirkt.

Zum anderen bedeuten alle chemischen Beanspruchungen eine Erschwerung von Färbeprozessen. Die äußere Beanspruchung der Haare durch chemische Stoffe aus einer Vielzahl unterschiedlicher Quellen stellt die Entwicklung kosmetischer Färbeprodukte vor Herausforderungen. Luft- und Wasserverunreinigungen wirken sich nachteilig auf Haut und Haare aus. Zu den wichtigsten Luftschadstoffen gehören polycyclische aromatische Kohlenwasserstoffe, flüchtige organische Verbindungen, Stickoxide (NOx), Partikel und Zigarettenrauch. Die Wirkung verschiedener Luftschadstoffe kann in Gegenwart anderer Luftschadstoffe und unter Einwirkung von UV-Strahlung verstärkt werden.

Ferner sind oft wechselnde Konsumentenwünsche hinsichtlich einer bestimmten Beschaffenheit der Haare mit einer wiederkehrenden chemischen Beanspruchung der Haare verbunden. Beispielsweise beanspruchen Haarfärbungen die Haare, aufgrund dessen eine besondere, intensive Pflege nötig sein kann.

Im Stand der Technik werden siliciumorganische Verbindungen aus der Gruppe der Silane beschrieben, die mindestens eine Hydroxygruppe und/oder hydrolysierbare Gruppe umfassen. Aufgrund der Anwesenheit der Hydroxygruppen und/oder hydrolysierbaren Gruppen handelt es sich bei den Silanen um reaktive Substanzen, die in Gegenwart von Wasser hydrolysieren bzw. oligomerisieren oder polymerisieren. Die durch Anwesenheit des Wassers initiierte Oligomerisierung oder Polymerisierung der Silane führt bei Anwendung auf einem keratinischen Material letztendlich zur Ausbildung eines Films, der eine Schutzwirkung entfalten kann. WO2017/102856A1 (L'OREAL) betrifft ein kosmetisches Mittel, welches zwei verschiedene Alkoxysilanen enthält. Das Mittel ermöglicht eine gute Formgebung der Haare und verbessert die Haltbarkeit der Haarfarben gegenüber Auswaschen durch Shampoos.

Die der vorliegenden Erfindung zugrunde liegende Aufgabe besteht in der Bereitstellung eines kosmetischen Produkts, das eine gute Formgebung der Haare ermöglicht und den Strukturschutz gegenüber chemischer Beanspruchung verbessert.

Diese Aufgabe wird gelöst durch ein kosmetisches Mittel zur Behandlung eines keratinischen Materials, umfassend a) mindestens eine organische Siliciumverbindung, wobei die mindestens eine organische Siliciumverbindung eine Verbindung der Formel (I) und mindestens eine organische Siliciumverbindung der Formel (IV) enthält,
wobei in der organischen Siliciumverbindung der Formel (I)

R₁R₂N-L-Si(OR₃)ₐ(R₄)_{b} (I),

- R₁, R₂ beide für ein Wasserstoffatom stehen,
- L für eine lineare, zweibindige C₁-C₆-Alkylengruppe, bevorzugt für eine Propylengruppe (-CH₂-CH₂-CH₂-) oder für eine Ethylengruppe (-CH₂-CH₂-), steht,
- R₃, R₄ unabhängig voneinander für eine Methylgruppe oder für eine Ethylgruppe stehen,
- a für die Zahl 3 steht und - b für die Zahl 0 steht, und wobei in der organischen Siliciumverbindung der Formel (IV) R₉Si(OR₁₀)ₖ(R₁₁)ₘ (IV),
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht,
- k für eine ganze Zahl von 1 bis 3 steht, und
- m für die ganze Zahl 3 - k steht,
und b) mindestens ein Polyacrylat-Copolymer, das gebildet wird aus den Monomeren Acrylsäure und Acrylamidopropyltrimoniumchlorid.

Unter einem keratinischen Material sind Haare, die Haut, die Nägel (wie beispielsweise Fingernägel und/oder Fußnägel) zu verstehen. Weiterhin fallen auch Wolle, Pelze und Federn unter die Definition des keratinischen Materials.

Bevorzugt werden unter einem keratinischen Material das menschliche Haar, die menschliche Haut und menschliche Nägel, insbesondere Finger- und Fußnägel, verstanden. Ganz besonders bevorzugt wird unter keratinischem Material das menschliche Haar, insbesondere Kopf- und/oder Barthaare, verstanden. Als ersten erfindungswesentlichen Bestandteil enthält das kosmetische Mittel zur Behandlung eines keratinischen Materials mindestens eine organische Siliciumverbindung, die ein bis drei Siliciumatome enthält. Bevorzugte organische Siliciumverbindungen werden ausgewählt aus Silanen mit einem, zwei oder drei Siliciumatomen, wobei die organische Siliciumverbindung eine oder mehrere Hydroxylgruppen und/oder hydrolysierbare Gruppen pro Molekül umfasst.

Organische Siliciumverbindungen, die alternativ auch als siliciumorganische Verbindungen bezeichnet werden, sind Verbindungen, die entweder eine direkte Silicium-Kohlenstoff-Bindung (Si-C) aufweisen oder in denen der Kohlenstoff über ein Sauerstoff-, Stickstoff- oder Schwefel-Atom an das Silicium-Atom geknüpft ist. Die organische Siliciumverbindungen sind Verbindungen, die ein bis drei Siliciumatome enthalten. Besonders bevorzugt enthalten die organische Siliciumverbindungen ein oder zwei Siliciumatome.

Die Bezeichnung Silan steht nach den IUPAC-Regeln für eine Stoffgruppe chemischer Verbindungen, die auf einem Silicium-Grundgerüst und Wasserstoff basieren. Bei organischen Silanen sind die WasserstoffAtome ganz oder teilweise durch organische Gruppen wie beispielsweise (substituierte) Alkylgruppen und/oder Alkoxygruppen ersetzt. In den organischen Silanen kann auch ein Teil der Wasserstoffatome durch Hydroxygruppen ersetzt sein.

Das Mittel zur Behandlung eines keratinischen Materials enthält mindestens eine organische Siliciumverbindung der Formel (I)

R₁R₂N-L-Si(OR₃)ₐ(R₄)_{b} (I),

wobei
- R₁, R₂ beide für ein Wasserstoffatom stehen,
- L für eine lineare, zweibindige C₁-C₆-Alkylengruppe, bevorzugt für eine Propylengruppe (-CH₂-CH₂-CH₂-) oder für eine Ethylengruppe (-CH₂-CH₂-), steht,
- R₃, R₄ unabhängig voneinander für eine Methylgruppe oder für eine Ethylgruppe stehen,
- a für die Zahl 3 steht und
- b für die Zahl 0 steht.

Das kosmetische Mittel kann eine weitere organische Siliciumverbindung der Formel (II) enthalten:

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- R5, R5', R5" unabhängig voneinander für ein Wasserstoffatom oder für eine C₁-C₆-Alkylgruppe stehen,
- R6, R6' und R6" unabhängig voneinander für eine C₁-C₆-Alkylgruppe stehen,
- A, A', A", A‴ und A"" unabhängig voneinander für eine lineare oder verzweigte, zweibindige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-alkyl-gruppe oder eine Gruppierung der Formel (III) stehen

   - (Aʺʺ)-Si(R₆")_{d}"(OR₅")_{c}" (III),
- c, für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
- mit der Maßgabe, dass mindestens einer der Reste aus e, f, g und h von 0 verschieden ist.

Die Substituenten R₁, R₂, R₃, R₄, R₅, R₅', R₅", R₆, R₆', R₆", R₇, R₈, L, A, A', A", A‴ und Aʺʺ in den Verbindungen der Formel (I) und (II) sind nachstehend beispielhaft erläutert:
Beispiele für eine C₁-C₆-Alkylgruppe sind die Gruppen Methyl, Ethyl, Propyl, Isopropyl, n-Butyl, s-Butyl und t-Butyl, n-Pentyl und n-Hexyl. Propyl, Ethyl und Methyl sind bevorzugte Alkylreste. Beispiele für eine C₂-C₆-Alkenylgruppe sind Vinyl, Allyl, But-2-enyl, But-3-enyl sowie Isobutenyl, bevorzugte C₂-C₆-Alkenylreste sind Vinyl und Allyl. Bevorzugte Beispiele für eine Hydroxy-C₁-C₆-alkylgruppe sind eine Hydroxymethyl-, eine 2-Hydroxyethyl-, eine 2-Hydroxypropyl, eine 3-Hydroxypropyl-, eine 4-Hydroxybutylgruppe, eine 5-Hydroxypentyl- und eine 6-Hydroxyhexylgruppe; eine 2-Hydroxyethylgruppe ist besonders bevorzugt. Beispiele für eine Amino-C₁-C₆-alkyl-gruppe sind die Aminomethylgruppe, die 2-Aminoethylgruppe, die 3-Aminopropylgruppe. Die 2-Aminoethylgruppe ist besonders bevorzugt. Beispiele für eine lineare zweibindige C₁-C₂₀-Alkylengruppe sind beispielsweise die Methylen-gruppe (-CH₂-), die Ethylengruppe (-CH₂-CH₂-), die Propylengruppe (-CH₂-CH₂-CH₂-) und die Butylengruppe (-CH₂-CH₂-CH₂-CH₂-). Die Propylengruppe (-CH₂-CH₂-CH₂-) ist besonders bevorzugt. Ab einer Kettenlänge von 3 C-Atomen können zweibindige Alkylengruppen auch verzweigt sein. Beispiele für verzweigte, zweibindige C₃-C₂₀-Alkylengruppen sind (-CH₂-CH(CH₃)-) und (-CH₂-CH(CH₃)-CH₂-).

In den organischen Siliciumverbindungen der Formel (I)

R₁R₂N-L-Si(OR₃)ₐ(R₄)_{b} (I),

stehen die Reste R₁ und R₂ unabhängig voneinander für ein Wasserstoffatom.

Der beste Schutz vor den negativen Auswirkungen von Wasser- und/oder Luftverschmutzungen ("Anti-Pollution"-Wirkung) und die beste Pflege von beanspruchtem Haar konnte erhalten werden, wenn das Mittel zur Behandlung eines keratinischen Materials mindestens eine organische Siliciumverbindung der Formel (I) enthält, in welcher die Reste R₃, R₄ unabhängig voneinander für eine Methylgruppe oder für eine Ethylgruppe stehen.

Besonders gut geeignete organische Siliciumverbindungen der Formel (I) sind
- (3-Aminopropyl)triethoxysilan 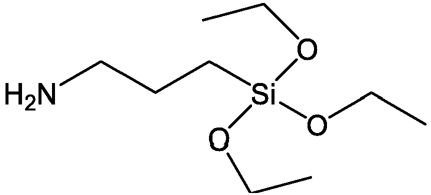
- (3-Aminopropyl)trimethoxysilan 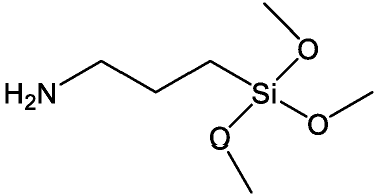
- 1-(3-Aminopropyl)silantriol 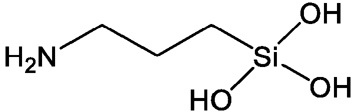
- (2-Aminoethyl)triethoxysilan 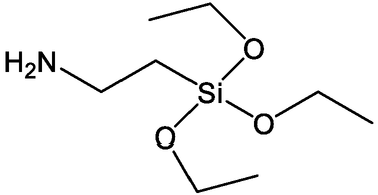
- (2-Aminoethyl)trimethoxysilan 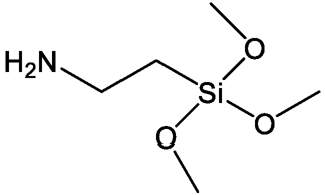
- 1-(2-Aminoethyl)silantriol

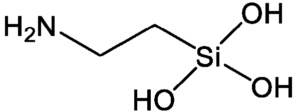

Die vorgenannten organische Siliciumverbindung der Formel (I) sind kommerziell erhältlich. (3-Aminopropyl)trimethoxysilan kann beispielsweise von Sigma-Aldrich käuflich erworben werden. Auch (3-Aminopropyl)triethoxysilan ist kommerziell bei der Firma Sigma-Aldrich erhältlich. Im Rahmen einer weiteren Ausführungsform enthält das Mittel zur Behandlung eines keratinischen Materials mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II).

Die siliciumorganischen Verbindungen der Formel (II) tragen jeweils an ihren beiden Enden die Siliciumhaltigen Gruppierungen (R₅O)_{c}(R₆)_{d}Si- und -Si(R₆')_{d'}(OR₅')_{c'}.

Im Mittelteil des Moleküls der Formel (II) befinden sich die Gruppierungen -(A)ₑ- und -[NR₇-(A')]_{f}- und -[O-(A")]_{g}- und -[NR₈-(A‴)]ₕ-. Hierbei kann jeder der Reste e, f, g und h unabhängig voneinander für die Zahl 0 oder 1 stehen, wobei die Maßgabe besteht, dass mindestens einer der Reste e, f, g und h von 0 verschieden ist. Mit anderen Worten enthält eine organischen Siliciumverbindung der Formel (II) mindestens eine Gruppierung aus der Gruppe aus -(A)- und -[NR₇-(A')]- und -[O-(A")]- und -[NR₈-(A‴)]-.

In den beiden endständigen Struktureinheiten (R₅O)_{c}(R₆)_{d}Si- und - Si(R₆')_{d'}(OR₅')_{c'} stehen die Reste R5, R5', R5" unabhängig voneinander für ein Wasserstoffatom oder für eine C₁-C₆-Alkylgruppe. Die Reste R6, R6' und R6" stehen unabhängig voneinander für eine C₁-C₆-Alkylgruppe.

Hierbei steht c für eine ganze Zahl von 1 bis 3, und d steht für die ganze Zahl 3 - c. Wenn c für die Zahl 3 steht, dann ist d gleich 0. Wenn c für die Zahl 2 steht, dann ist d gleich 1. Wenn c für die Zahl 1 steht, dann ist d gleich 2.

Analog steht c' für eine ganze Zahl von 1 bis 3, und d' steht für die ganze Zahl 3 - c'. Wenn c' für die Zahl 3 steht, dann ist d' gleich 0. Wenn c' für die Zahl 2 steht, dann ist d' gleich 1. Wenn c' für die Zahl 1 steht, dann ist d' gleich 2.

Eine sehr hohe Anti-Pollution-Wirkung des Mittels zur Behandlung eines keratinischen Materials konnte erhalten werden, wenn die Reste c und c' beide für die Zahl 3 stehen. In diesem Fall stehen d und d' beide für die Zahl 0.

In einer weiteren bevorzugten enthält das Mittel zur Behandlung eines keratinischen Materials mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- R5 und R5' unabhängig voneinander für eine Methylgruppe oder eine Ethylgruppe stehen,
- c und c' beide für die Zahl 3 stehen und
- d und d' beide für die Zahl 0 stehen.

Wenn c und c' beide für die Zahl 3 stehen und d und d' beide für die Zahl 0 stehen, entsprechen die organischen Siliciumverbindung der Formel (IIa)

(R₅O)₃Si-(A)ₑ-[NR₇-(A')]_{f}-[O-(A")]_{g}-[NR₈-(A‴)]ₕ-Si(OR₅')₃ (IIa).

Die Reste e, f, g und h können unabhängig voneinander für die Zahl 0 oder 1 stehen, wobei mindestens ein Rest aus e, f, g und h von null verschieden ist. Durch die Kürzel e, f, g und h wird demnach definiert, welche der Gruppierungen -(A)ₑ- und -[NR₇-(A')]_{f}- und -[O-(A")]_{g}- und -[NR₈-(A‴)]ₕ- sich im Mittelteil der organischen Siliciumverbindung der Formel (II) befinden.

In diesem Zusammenhang hat sich die Anwesenheit bestimmter Gruppierungen als besonders vorteilhaft im Hinblick auf die Erhöhung der "Anti-Pollution"-Wirkung erwiesen. Besonders gute Ergebnisse konnten erhalten werden, wenn mindestens zwei der Reste e, f, g und h für die Zahl 1 stehen. Ganz besonders bevorzugt stehen e und f beide für die Zahl 1. Weiterhin ganz besonders bevorzugt stehen g und h beide für die Zahl 0.

Wenn e und f beide für die Zahl 1 stehen und g und h beide für die Zahl 0 stehen, entsprechen die organischen Siliciumverbindung der Formel (IIb)

(R₅O)_{c}(R₆)_{d}Si-(A)-[NR₇-(A')]-Si(R₆')_{d'}(OR₅')_{c'} (IIb).

Die Reste A, A', A", A‴ und A"" stehen unabhängig voneinander für eine lineare oder verzweigte, zweibindige C₁-C₂₀-Alkylengruppe. Bevorzugt stehen die Reste A, A', A", A‴ und Aʺʺ unabhängig voneinander für eine lineare, zweibindige C₁-C₂₀-Alkylengruppe. Weiter bevorzugt stehen die Reste A, A', A", A‴ und Aʺʺ unabhängig voneinander für eine lineare zweibindige C₁-C₆-Alkylengruppe. Besonders bevorzugt stehen die Reste A, A', A", A‴ und A"" unabhängig voneinander für eine Methylengruppe (-CH₂-), eine Ethylengruppe (-CH₂-CH₂-), eine Propylengruppe (-CH₂-CH₂-CH₂-) oder eine Butylengruppe (-CH₂-CH₂-CH₂-CH₂-). Ganz besonders bevorzugt stehen die Reste A, A', A", A‴ und A"" für eine Propylengruppe (-CH₂-CH₂-CH₂-).

Wenn der Rest f für die Zahl 1 steht, dann enthält die organische Siliciumverbindung der Formel (II) eine strukturelle Gruppierung -[NR₇-(A')]-.

Wenn der Rest h für die Zahl 1 steht, dann enthält die organische Siliciumverbindung der Formel (II) eine strukturelle Gruppierung -[NR₈-(A‴)]-.

Hierbei stehen die Reste R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-Alkylgruppe oder eine Gruppierung der Formel (III)

- (Aʺʺ)-Si(Rₑ")_{d}"(OR₅")_{c}" (III).

Ganz besonders bevorzugt stehen die Reste R₇ und R₈ unabhängig voneinander für ein Wasserstoffatom, eine Methylgruppe, eine 2-Hydroxyethylgruppe, eine 2-Alkenylgruppe, eine 2-Aminoethylgruppe oder für eine Gruppierung der Formel (III).

Wenn der Rest f für die Zahl 1 steht und der Rest h für die Zahl 0 steht, enthält die organische Siliciumverbindung die Gruppierung [NR₇-(A')], aber nicht die Gruppierung -[NR₈-(A‴)]. Steht nun der Rest R7 für eine Gruppierung der Formel (III), so enthält das Mittel zur Behandlung eines keratinischen Materials eine organische Siliciumverbindung mit 3 reaktiven Silan-Gruppen.

In einer weiteren bevorzugten enthält das Mittel zur Behandlung eines keratinischen Materials mindestens eine organische Siliciumverbindung der Formel (II)

(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),

wobei
- e und f beide für die Zahl 1 stehen,
- g und h beide für die Zahl 0 stehen,
- A und A' unabhängig voneinander für eine lineare, zweibindige C₁-C₆-Alkylengruppe stehen
   und
- R7 für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

In einer weiteren bevorzugten Ausführungsform enthält das Mittel zur Behandlung eines keratinischen Materials mindestens eine organische Siliciumverbindung der Formel (II), wobei
- e und f beide für die Zahl 1 stehen,
- g und h beide für die Zahl 0 stehen,
- A und A' unabhängig voneinander für eine Methylengruppe (-CH₂-), eine Ethylengruppe (-CH₂-CH₂-) oder eine Propylengruppe (-CH₂-CH₂-CH₂) stehen,
   und
- R₇ für ein Wasserstoffatom, eine Methylgruppe, eine 2-Hydroxyethylgruppe, eine 2-Alkenylgruppe, eine 2-Aminoethylgruppe oder für eine Gruppierung der Formel (III) steht.

Zur Lösung der Aufgabenstellung gut geeignete organische Siliciumverbindungen der Formel (II) sind
- 3-(Trimethoxysilyl)-N-[3-(trimethoxysilyl)propyl]-1-propanamin 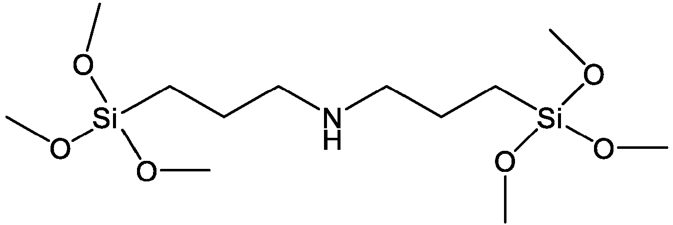
- 3-(Triethoxysilyl)-N-[3-(triethoxysilyl)propyl]-1-propanamin 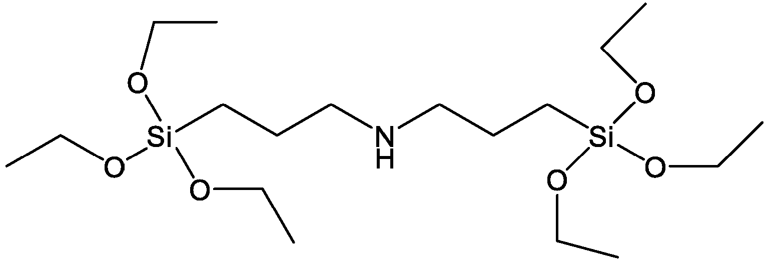
- N-Methyl-3-(trimethoxysilyl)-N-[3-(trimethoxysilyl)propyl]-1-propanamin 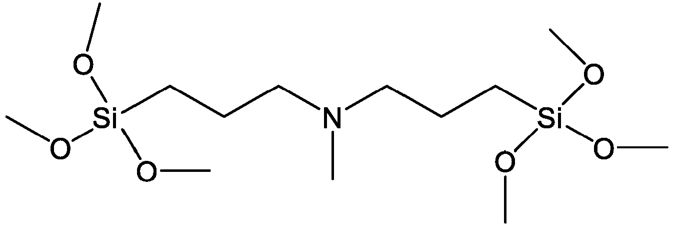
- N-Methyl-3-(triethoxysilyl)-N-[3-(triethoxysilyl)propyl]-1-propanamin 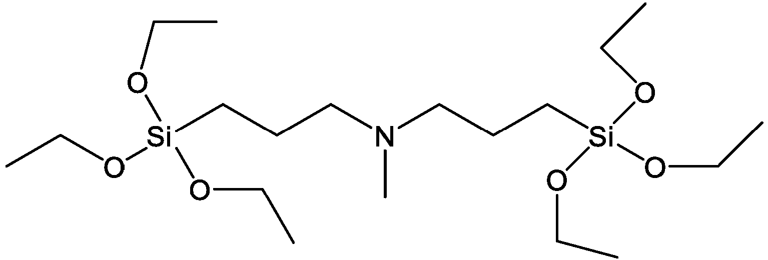
- 2-[Bis[3-(trimethoxysilyl)propyl]amino]-ethanol 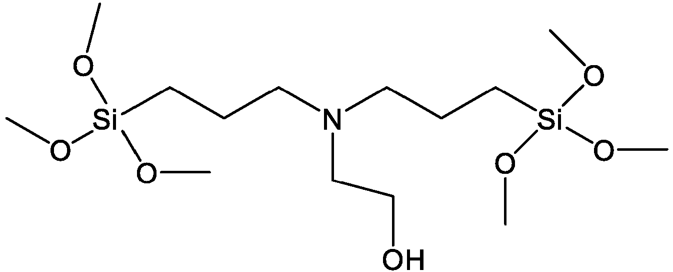
- 2-[Bis[3-(triethoxysilyl)propyl]amino]-ethanol 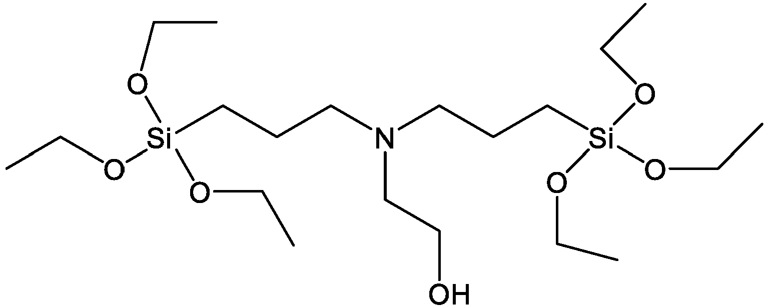
- 3-(Trimethoxysilyl)-N,N-bis[3-(trimethoxysilyl)propyl]-1-propanamin 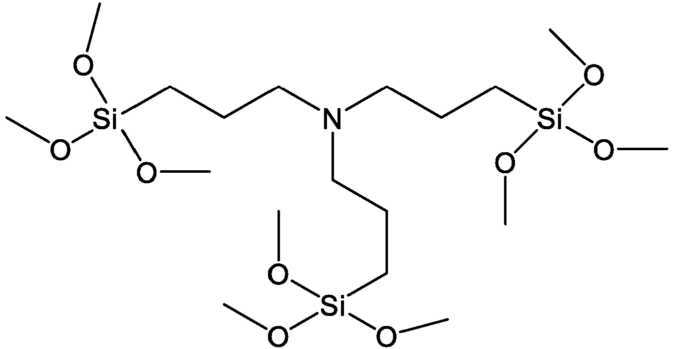
- 3-(Triethoxysilyl)-N,N-bis[3-(triethoxysilyl)propyl]-1-propanamin 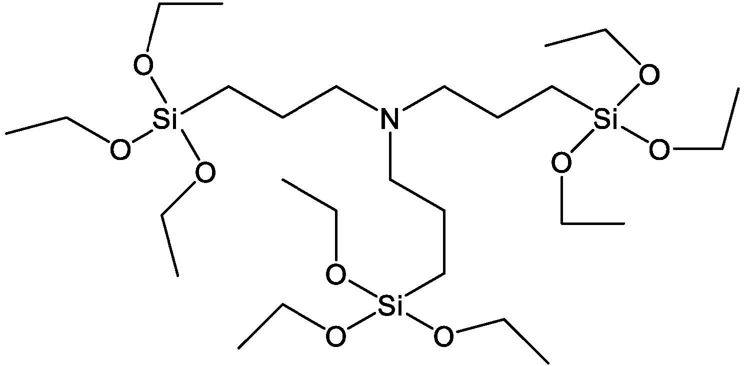
- N1,N1-Bis[3-(trimethoxysilyl)propyl]-1,2-ethanediamin, 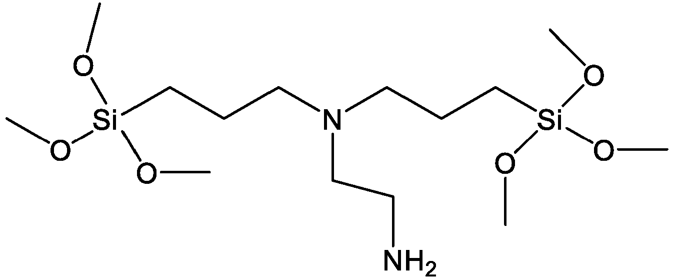
- N1,N1-Bis[3-(triethoxysilyl)propyl]-1,2-ethanediamin, 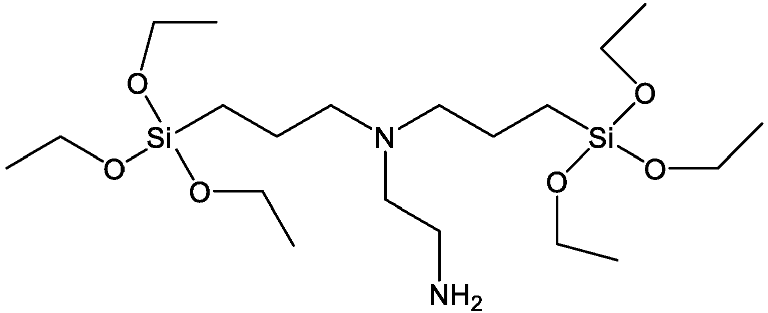
- N,N-Bis[3-(trimethoxysilyl)propyl]-2-propen-1-amin 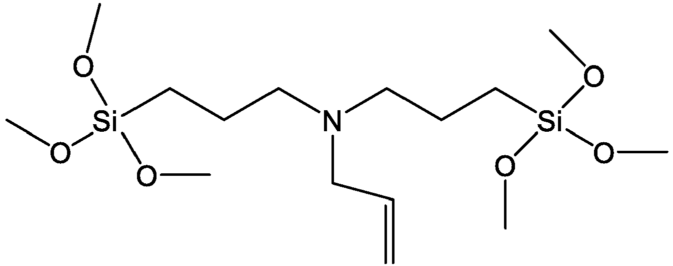
- N,N-Bis[3-(triethoxysilyl)propyl]-2-propen-1-amin

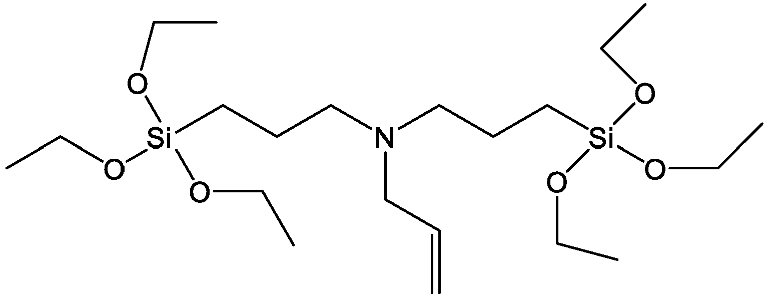

Die vorgenannten organische Siliciumverbindung der Formel (II) sind kommerziell erhältlich.

Bis(trimethoxysilylpropyl)amin mit der CAS-Nummer 82985-35-1 kann beispielsweise von Sigma-Aldrich käuflich erworben werden.

Bis[3-(triethoxysilyl)propyl]amin, auch bezeichnet als 3-(Triethoxysilyl)-N-[3-(triethoxysilyl)propyl]-1-propanamin, mit der CAS-Nummer 13497-18-2 kann zum Beispiel von Sigma-Aldrich käuflich erworben werden oder ist im Handel unter der Produktbezeichnung Dynasylan 1122 von Evonik erhältlich. N-Methyl-3-(trimethoxysilyl)-N-[3-(trimethoxysilyl)propyl]- 1-propanamin wird alternativ auch als Bis(3-trimethoxysilylpropyl)-N-methylamin bezeichnet und kann bei Sigma-Aldrich oder Fluorochem kommerziell erworben werden.
3-(Triethoxysilyl)-N,N-bis[3-(triethoxysilyl)propyl]-1-propanamin mit der CAS-Nummer 18784-74-2 kann beispielsweise von Fluorochem oder Sigma-Aldrich käuflich erworben werden.

Es sich ebenfalls als vorteilhaft herausgestellt, wenn das auf dem Haar angewendete Mittel zur Behandlung eines keratinischen Materials mindestens eine organische Siliciumverbindung der Formel (IV) enthält

R₉Si(OR₁₀)ₖ(R₁₁)ₘ (IV).

Die Verbindungen der Formel (IV) sind organische Siliciumverbindungen, die aus Silanen mit einem, zwei oder drei Siliciumatomen ausgewählt sind, wobei die organische Siliciumverbindung eine oder mehrere Hydroxylgruppen und/oder hydrolysierbare Gruppen pro Molekül umfasst.

Das bzw. die organischen Siliciumverbindungen der Formel (IV) können auch als Silane vom Typ der Alkylalkoxysilane oder der Alkylhydroxysilane bezeichnet werden,

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

Das Mittel zur Behandlung eines keratinischen Materials enthält

zusätzlich zu der oder den organischen Siliciumverbindungen der Formel (I) mindestens eine weitere organische Siliciumverbindung der Formel (IV)

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

Das Mittel zur Behandlung eines keratinischen Materials enthält zusätzlich zu den organischen Siliciumverbindungen der Formel (I) mindestens eine weitere organische Siliciumverbindung der Formel (IV)

R₉Si(OR₁₀)ₖ(R₁₁)ₘ (IV),

wobei
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht.

In den organischen Siliciumverbindungen der Formel (IV) steht der Rest R₉ für eine C₁-C₁₂-Alkylgruppe.

Diese C₁-C₁₂-Alkylgruppe ist gesättigt und kann linear oder verzweigt sein. Bevorzugt steht R9 für eine lineare C₁-C₈-Alkylgruppe. Bevorzugt steht R₉ für eine Methylgruppe, eine Ethylgruppe, eine n-Propylgruppe, eine n-Butylgruppe, eine n-Pentylgruppe, eine n-Hexylgruppe, eine n-Octylgruppe oder eine n-Dodecylgruppe. Besonders bevorzugt steht R₉ für eine Methylgruppe, eine Ethylgruppe oder eine n-Octylgruppe.

In den organischen Siliciumverbindungen der Formel (IV) steht der Rest R₁₀ für ein Wasserstoffatom oder eine C₁-C₆-Alkylgruppe. Besonders bevorzugt steht R₁₀ für eine Methylgruppe oder für eine Ethylgruppe. In den organischen Siliciumverbindungen der Formel (IV) steht der Rest R₁₁ für eine C₁-C₆-Alkylgruppe. Besonders bevorzugt steht R₁₁ für eine Methylgruppe oder für eine Ethylgruppe.

Weiterhin steht k für eine ganze Zahl von 1 bis 3, und m steht für die ganze Zahl 3 - k. Wenn k für die Zahl 3 steht, dann ist m gleich 0. Wenn k für die Zahl 2 steht, dann ist m gleich 1. Wenn k für die Zahl 1 steht, dann ist m gleich 2.

Eine sehr hohe "Anti-Pollution"-Wirkung konnte erhalten werden, wenn das Mittel zur Behandlung eines keratinischen Materials mindestens eine organische Siliciumverbindung der Formel (IV) enthält, in welcher der Rest k für die Zahl 3 steht. In diesem Fall steht der Rest m für die Zahl 0.

Zur Lösung der Aufgabenstellung besonders gut geeignete organische Siliciumverbindungen der Formel (IV) sind
- Methyltrimethoxysilan 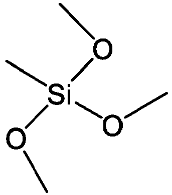
- Methyltriethoxysilan 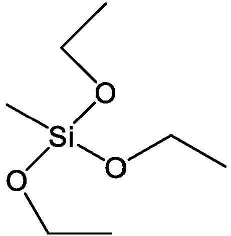
- Ethyltrimethoxysilan 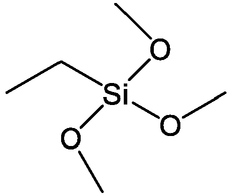
- Ethyltriethoxysilan 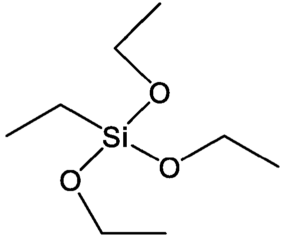
- n-Hexyltrimethoxysilan 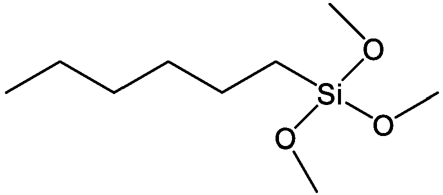
- n-Hexyltriethoxysilan 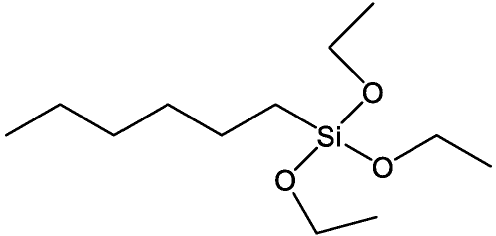
- n-Octyltrimethoxysilan 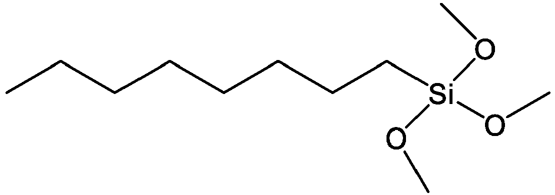
- n-Octyltriethoxysilan 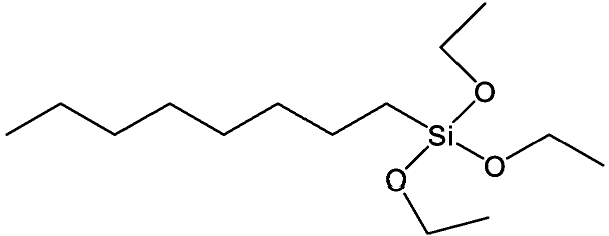
- n-Dodecyltrimethoxysilan und/oder 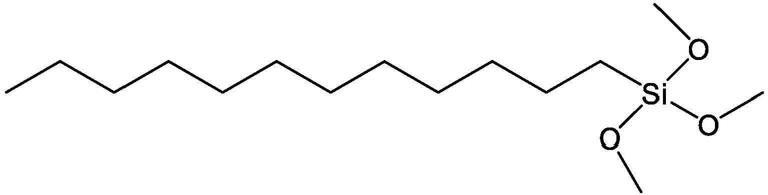
- n-Dodecyltriethoxysilan 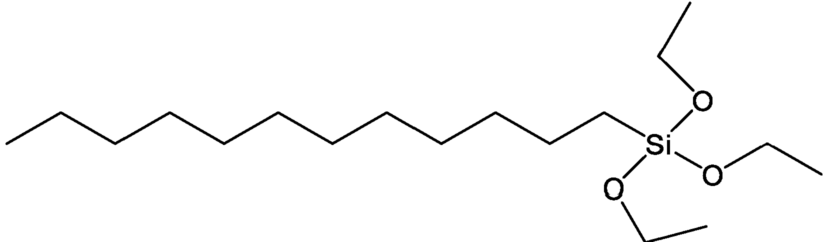
sowie Propyltrimethoxysilan und Propyltriethoxysilan.

Bei den zuvor beschriebenen organischen Siliciumverbindungen handelt es sich um reaktive Verbindungen.

Es hat sich herausgestellt, dass auf dem keratinischen Material auch dann besonders stabile und gleichmäßige Filme erhalten werden konnten, wenn das Mittel mindestens eine organische Siliciumverbindung eine Verbindung der Formel (I) und mindestens eine organische Siliciumverbindung der Formel (IV) enthält.

In einer bevorzugten Ausführungsform ist ein Mittel dadurch gekennzeichnet, dass es mindestens eine organische Silicumverbindungen der Formel (I) und mindestens eine organische Silicumverbindung der Formel (IV) enthält.

In einer explizit ganz besonders bevorzugten Ausführungsform ist ein Mittel dadurch gekennzeichnet, dass es mindestens eine organische Silicumverbindungen der Formel (I) enthält, welche aus der Gruppe aus (3-Aminopropyl)triethoxysilan und (3-Aminopropyl)trimethoxysilan ausgewählt ist, und zusätzlich mindestens eine organische Silicumverbindungen der Formel (IV) enthält, welche aus der Gruppe bestehend aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan und Hexyltriethoxysilan ausgewählt ist.

In einer weiteren bevorzugten Ausführungsform ist ein Mittel dadurch gekennzeichnet, dass das Mittelbezogen auf das Gesamtgewicht des Mittels - enthält:
- 0,5 bis 5 Gew.-% mindestens einer ersten organischen Siliciumverbindung, die ausgewählt ist aus der Gruppe aus (3-Aminopropyl)trimethoxysilan, (3-Aminopropyl)triethoxysilan, (2-Aminoethyl)trimethoxysilan, (2-Aminoethyl)triethoxysilan, (3-Dimethylaminopropyl)trimethoxysilan, (3-Dimethylaminopropyl)triethoxysilan (2-Dimethylaminoethyl)trimethoxysilan und (2-Dimethylaminoethyl)triethoxysilan, und
- 3,2 bis 10 Gew.-% mindestens einer zweiten organischen Siliciumverbindung, die ausgewählt ist aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octyldecyltrimethoxysilan und Octyldecyltriethoxysilan.

Bereits der Zusatz geringer Wassermengen führt bei organischen Siliciumverbindungen mit mindestens einer hydrolysierbaren Gruppe zur Hydrolyse. Die Hydrolyseprodukte und/oder organischen Siliciumverbindungen mit mindestens einer Hydroxygruppe können in einer Kondensationsreaktion miteinander reagieren. Aus diesem Grund können sowohl die siliciumorganischen Verbindungen mit mindestens einer hydrolysierbaren Gruppe als auch deren Hydrolyse- und/oder Kondensationsprodukte in dem Mittel enthalten sein. Bei Verwendung von siliciumorganischen Verbindungen mit mindestens einer Hydroxylgruppe können sowohl die organischen Siliciumverbindungen mit mindestens einer Hydroxylgruppe als auch deren Kondensationsprodukte in dem Mittel enthalten sein.

Unter einem Kondensationsprodukt wird ein Produkt verstanden, dass durch Reaktion von mindestens zwei organischen Siliciumverbindungen mit jeweils mindestens einer Hydroxylgruppen oder hydrolysierbaren Gruppen pro Molekül unter Abspaltung von Wasser und/oder unter Abspaltung von einem Alkanol entsteht. Die Kondensationsprodukte können beispielsweise Dimere, aber auch Trimere oder Oligomere sein, wobei die Kondensationsprodukte mit den Monomeren im Gleichgewicht stehen. Abhängig von der eingesetzten oder in der Hydrolyse verbrauchten Wassermenge verschiebt sich das Gleichgewicht von monomerer organischen Siliciumverbindungen zu Kondensationsprodukt.

Im Rahmen der vorliegenden Erfindung sind Angaben in Gew.-% - falls nicht anders angegeben - immer bezogen auf das Gesamtgewicht des kosmetischen Mittels.

Als erfindungswesentlichen Bestandteil enthält das kosmetische Mittel zur Behandlung eines keratinischen Materials mindestens ein Polyacrylat-Copolymer, das aus den Monomeren Acrylsäure und

Acrylamidopropyltrimoniumchlorid gebildet wird. Das mindestens eine Polyacrylat-Copolymer wird somit erfindungsgemäß aus den folgenden Monomeren: 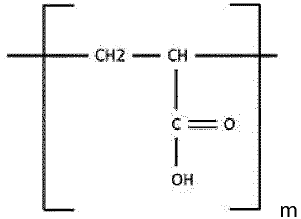 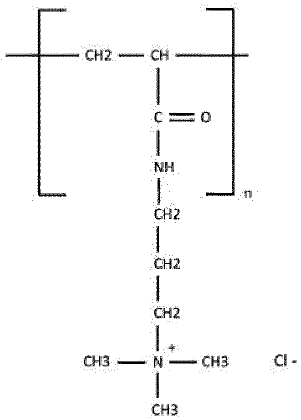 gebildet, in denen m und n für die Anzahl der Comonomeren im Polymer stehen, wobei das Verhältnis von m zu n zwischen 90 zu 10 bis 10 zu 90, bevorzugt zwischen 75 zu 25 bis 25 zu 75, bevorzugter zwischen 60 zu 40 und 40 zu 60 liegt.

Das Polyacrylat-Copolymer umfasst gemäß einer bevorzugten Ausführungsform der vorliegenden Erfindung weitere, von diesen Monomeren verschiedene Monomereinheiten.

Im Zuge der zu dieser Erfindung führenden Arbeiten hat sich herausgestellt, dass es zur Erzielung von vorteilhaften Eigenschaften hinsichtlich des Auswaschschutzes gefärbter Haare bei gleichzeitigem Strukturschutz von Vorteil ist, wenn die organische Siliciumverbindungen, insbesondere 3-(Triethoxysilyl)-N-[3-(triethoxysilyl)propyl]-1-propanamin, d.h. ein Bis(triethoxysilylpropyl)amin, und/oder (3-Aminopropyl)triethoxysilan, d.h. ein Aminopropyltriethoxysilan (AMEO), mit dem Polyacrylat-Copolymer kombiniert wird.

Ein besonders bevorzugtes Produkt, das als zwingende Komponente eingesetzt werden kann, ist das Produkt W 37194 von der Firma Giovanni Bozzetto S.p. Das Polyacrylat-Copolymer ist unter der INCI-Bezeichnung Acrylamidopropyltrimonium Chloride/Acrylates Copolymer bekannt.

Die Kombination aus der organischen Siliciumverbindung, die ein bis drei Siliciumatome enthält, mit dem Polyacrylat-Copolymer bildet eine Schicht auf dem Haar. Dadurch wird gewährleistet, dass das Haar hydrophobisiert wird, was zur Reduktion von Frizz führt. Darüber hinaus wird der Auswaschschutz gefärbter Haare verbessert.

Gemäß einer bevorzugten Ausführungsform der vorliegenden Erfindung beträgt die Menge an Polyacrylat-Copolymer in dem kosmetischen Mittel 0,01 bis 10 Gew.-%, bevorzugt von 0,25 bis 8 Gew.-%, bevorzugter von 0,5 bis 6 Gew.-%, bezogen auf das Gesamtgewicht des kosmetischen Mittels.

Im Folgenden werden weitere Bestandteile des kosmetischen Mittels beschrieben, die neben den zuvor beschriebenen zwingenden und optionalen Inhaltstoffen in den Mitteln enthalten sein können.

Es kann bevorzugt sein, dass das Mittel zur Behandlung eines keratinischen Materials ferner 0,001 bis 20 Gew.-% mindestens einer quaternären Verbindung umfasst. Dies gilt insbesondere für Mittel, die zusätzlich Pflegeeigenschaften dem keratinischen Material verleihen.

Es ist bevorzugt, dass die mindestens eine quaternäre Verbindung ausgewählt aus mindestens einer der Gruppen bestehend aus
i) der Monoalkylquats und/oder
ii) der Esterquats und/oder
iii) der quaternären Imidazoline der Formel (Tkat2), 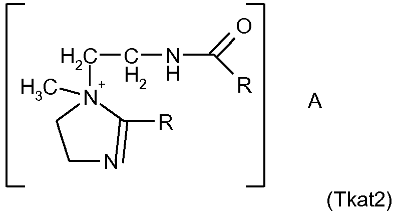 in welcher die Reste R unabhängig voneinander jeweils für einen gesättigten oder ungesättigten, linearen oder verzweigten Kohlenwasserstoffrest mit einer Kettenlänge von 8 bis 30 Kohlenstoffatomen und A für ein physiologisch verträgliches Anion steht, und/oder
iv) der Amidoamine und/oder kationisierten Amidoamine und/oder
v) quaternisierten Cellulose-Derivaten, insbesondere Polyquaternium 10, Polyquaternium-24, Polyquaternium-27, Polyquaternium-67, Polyquaternium-72, und/oder
vi) kationischen Alkylpolyglycosiden und/oder
vii) kationisiertem Honig und/oder
viii) kationischen Guar-Derivaten und/oder
ix) Chitosan und/oder
x) quaterniertem Polyvinylalkohol, sowie Mischungen hiervon.

Ferner können festigende Verbindungen, vorzugsweise ausgewählt aus der Gruppe bestehend aus Wachsen und/oder weiteren synthetischen Polymeren in dem kosmetischen Mittel enthalten sein. D.h. ergänzend können Wachse und/oder weitere synthetische Polymere als festigende Verbindungen eingesetzt werden. Idealerweise ergeben die Polysaccharide und die weiteren festigenden Verbindungen bei der Anwendung auf dem keratinischen Material einen Film, der einerseits der Frisur einen starken Halt verleiht, andererseits aber hinreichend flexibel ist, um bei Beanspruchung nicht zu brechen.

Beispielsweise können Polycarbonsäuren in dem kosmetischen Mittel als Filmbildner eingesetzt werden. Diese lagern sich auf den Haaren ab und begründen eine temporäre Verformbarkeit der Haare. Der Filmbildner kann ein Homo- oder Copolymer sein, das aus Itaconsäure abgeleitet ist. Wenn der Filmbildner ausschließlich in Form von polymerisierter Itaconsäure und/oder einem Salz der Itaconsäure vorliegt, bildet der Filmbildner ein Homopolymer. Ein Beispiel eines solchen Polymers ist PVP/VA/Itaconic Acid Copolymer (INCI).

Die weiteren synthetischen Polymere lassen sich in kationische, anionische, nichtionische und amphotere festigende Polymere unterteilen.

Geeignete synthetische Polymere umfassen beispielsweise Polymere mit den folgenden INCI-Bezeichnungen: Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/Hydroxyesters Acrylates Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis- Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer. Ebenso geeinet sind Celluloseether, wie Hydroxypropylcellulose, Hydroxyethylcellulose und Methylhydroxypropylcellulose.

Bevorzugt umfasst die festigende Verbindung ein Vinylpyrrolidon-haltiges Polymer. Besonders bevorzugt umfasst die festigende Verbindung ein Polymer ausgewählt aus der Gruppe bestehend aus Polyvinylpyrrolidon (PVP), Vinylpyrrolidon-Vinylacetat-Copolymer (VP/VA Coplymer), Vinyl Caprolactam/VP/Dimethylaminoethyl Methacrylate Copolymer (INCI), VP/DMAPA Acrylates Copolymer (INCI) und Mischungen daraus.

Entsprechend ist es besonders bevorzugt, dass die festigende Verbindung ein synthetisches Polymer ausgewählt aus der Gruppe bestehend aus Polyvinylpyrrolidon (PVP), Vinylpyrrolidon-Vinylacetat-Copolymer (VP/VA Coplymer), Vinyl Caprolactam/VP/Dimethylaminoethyl Methacrylate Copolymer (INCI), VP/DMAPA Acrylates Copolymer (INCI), Octylacrylamide/Acrylates/Butylaminoethyl Methacrylate Copolymer (INCI) und Mischungen daraus umfasst.

Ferner kann als ein festigendes Polymer ein Octylacrylamide/Acrylates/Butylaminoethyl Methacrylate Copolymer (INCI) in dem kosmetischen Mittel enthalten. Dieses Copolymer wird unter den Bezeichnungen "Amphomer^{®}", "Amphomer^{®} HC", "Amphomer^{®} 4961", "Amphomer^{®} EDGE" und "Amphomer^{®} LV 71" in verschiedenen Abwandlungen von Akzo Nobel vertrieben wird.

Ferner können in bevorzugten Ausführungsformen Polysaccharide in den kosmetischen Mitteln enthalten sein. Gemäß einigen Ausführungsformen handelt es sich bei den bevorzugt enthaltenen Polysacchariden um Stärke, thermisch und/oder mechanisch behandelte Stärke, oxidativ, hydrolytisch oder oxidierte enzymatisch abgebaute Stärken, oxidierte hydrolytisch oder oxidierte enzymatisch abgebaute Stärken sowie chemisch modifizierte Stärken. In diesem Zusammenhang sind prinzipiell alle Stärken geeignet. Gemäß einigen Ausführungsformen kann es sich beispielsweise, ohne Einschränkung, um Stärke aus Mais, Weizen, Reis, Erbsen, Gerste, Roggen, Maniok, Tapioka, Süßkartoffeln oder Kartoffeln handeln. In einigen Ausführungsformen wird vorzugsweise native Stärke eingesetzt. Als native Stärke wird Stärke bezeichnet, wie sie aus natürlichen, beispielsweise den vorgenannten, Quellen zugänglich ist. Native Stärke ist ein handelsübliches Produkt und damit leicht zugänglich.

Gemäß einer Ausführungsform handelt es sich bei dem Polysaccharid um Mais-, Weizen-, Reis-, Erbsen-, Gerste-, Roggen-, Maniok-, Tapioka-, Süßkartoffel- oder Kartoffelstärke. Gemäß einer Ausführungsform handelt es sich bei dem Polysaccharid um die entsprechende native Stärke der vorgenannten Art. Auch Mischungen der vorgenannten sind geeignet. Gemäß einer Ausführungsform handelt es sich bei dem Polysaccharid vorzugsweise um Maisstärke, insbesondere native Maisstärke. Am meisten bevorzugt ist die Maisstärke Collamyl 8412 der Firma Agrabne Wien.

Um den unterschiedlichen Anforderungen an Mittel zur Behandlung eines keratinischen Materials in Form eines Mittels zum temporären Umformen eines keratinischen Materials (= Stylingmittel) gerecht zu werden, sind als festigende Verbindungen bereits eine Vielzahl von synthetischen Polymeren entwickelt worden, die in einem Mittel zur Behandlung eines keratinischen Materials zur Anwendung kommen können.

Das Mittel zur Behandlung eines keratinischen Materials kann insbesondere ein Mittel zur temporären Umformung eines keratinischen Materials, ein Mittel zur Reinigung eines keratinischen Materials, ein Mittel zur Pflege eines keratinischen Materials und/oder ein Mittel zur Pflege und Reinigung eines keratinischen Materials umfassen.

Die kosmetische Zusammensetzung kann zusätzlich oder alternativ zu einem synthetischen Polymer mindestens ein natürliches oder synthetisches Wachs, welches einen Schmelzpunkt von über 37 °C aufweist, als festigende Verbindung enthalten.

Als natürliche oder synthetische Wachse können feste Paraffine oder Isoparaffine, Pflanzenwachse wie Candelillawachs, Carnaubawachs, Espartograswachs, Japanwachs, Korkwachs, Zuckerrohrwachs, Ouricurywachs, Montanwachs, Sonnenblumenwachs, Fruchtwachse und tierische Wachse, wie zum Beispiel Bienenwachse und andere Insektenwachse, Walrat, Schellackwachs, Wollwachs und Bürzelfett, weiterhin Mineralwachse, wie zum Beispiel Ceresin und Ozokerit oder die petrochemischen Wachse, wie zum Beispiel Petrolatum, Paraffinwachse, Microwachse aus Polyethylen oder Polypropylen und Polyethylenglycolwachse eingesetzt werden. Es kann vorteilhaft sein, hydrierte oder gehärtete Wachse einzusetzen. Weiterhin sind auch chemisch modifizierte Wachse, insbesondere die Hartwachse, zum Beispiel Montanesterwachse, Sasolwachse und hydrierte Jojobawachse, einsetzbar.

Weiterhin geeignet sind die Triglyceride gesättigter und gegebenenfalls hydroxylierter C16-30-Fettsäuren, wie zum Beispiel gehärtete Triglyceridfette (hydriertes Palmöl, hydriertes Kokosöl, hydriertes Rizinusöl), Glyceryltribehenat oder Glyceryltri-12-hydroxystearat.

Die Wachskomponenten können auch aus der Gruppe der Ester aus gesättigten, unverzweigten Alkancarbonsäuren einer Kettenlänge von 22 bis 44 C-Atomen und gesättigten, unverzweigten Alkoholen einer Kettenlänge von 22 bis 44 C-Atomen ausgewählt werden, sofern die Wachskomponente oder die Gesamtheit der Wachskomponenten bei Raumtemperatur fest sind. Auch Silikonwachse, zum Beispiel Stearyltrimethylsilan/Stearylalkohol sind gegebenenfalls vorteilhaft.

Natürliche, chemisch modifizierte und synthetische Wachse können alleine oder in Kombination eingesetzt werden. Es können somit auch mehrere Wachse eingesetzt werden. Weiterhin ist auch eine Reihe von Wachsmischungen, ggf. in Abmischung mit weiteren Zusätzen, im Handel erhältlich. Die unter den Bezeichnungen "Spezialwachs 7686 OE" (eine Mischung aus Cetylpalmitat, Bienenwachs, mikrokristallinem Wachs und Polyethylen mit einem Schmelzbereich von 73-75 °C; Hersteller: Kahl & Co), Polywax^{®} GP 200 (eine Mischung von Stearylalkohol und Polyethylenglykolstearat mit einem Schmelzpunkt von 47-51 °C; Hersteller: Croda) und "Weichceresin^{®} FL 400" (ein Vaseline/Vaselinöl/Wachs-Gemisch mit einem Schmelzpunkt von 50-54 °C; Hersteller: Parafluid Mineralölgesellschaft) sind Beispiele für einsetzbare Mischungen.

Bevorzugt ist das Wachs ausgewählt aus Carnaubawachs (INCI: Copernicia Cerifera Cera) Bienenwachs (INCI: Beeswax), Petrolatum (INCI), mikrokristallinem Wachs und insbesondere Gemischen daraus.

Bevorzugte Mischungen umfassen die Kombination von Carnaubawachs (INCI: Copernicia Cerifera Cera), Petrolatum und mikrokristallinem Wachs oder die Kombination von Bienenwachs (INCI: Beeswax) und Petrolatum.

Das Wachs oder die Wachskomponenten sollten bei 25 °C fest sein und sollen im Bereich von > 37 °C schmelzen

Das Mittel zur Behandlung eines keratinischen Materials enthält eine festigende Verbindung vorzugsweise in einer Gesamtmenge von 0,5 bis 50 Gew.-%, bevorzugt 1 bis 40 Gew.-%, weiter bevorzugt 1,5 bis 30 Gew.-%, noch mehr bevorzugt 2 bis 25 Gew.-%, bezogen auf das Gesamtgewicht der kosmetischen Zusammensetzung.

Weitere geeignete Inhaltsstoffe umfassen nichtionische Polymere, anionische Polymere, (weitere) kationische Polymere, Wachse, Proteinhydrolysate, Aminosäuren, Oligopetide, Vitamine, Provitamine, Vitaminvorstufen, Betaine, Biochinone, Purin(derivate), Pflegestoffe, Pflanzenextrakte, Silikone, Esteröle, UV-Lichtschutzfilter, Verdickungsmittel, Elektrolyte, pH-Stellmittel, Quellmittel, Farbstoffe, Antischuppenwirkstoffe, Komplexbildner, Trübungsmittel, Perlglanzmittel, Pigmente, Stabilisierungsmittel, Treibmittel, Antioxidantien, Parfümöle und/oder Konservierungsmittel.

In den Ausführungsformen 1 bis 48 werden organischen Siliciumverbindungen mit dem bevorzugten Polyacrylat-Copolymer miteinander kombiniert. Die Kombinationen der organischen Siliciumverbindungen der Formel (I) und der Formel (IV) mit dem Polyacrylat-Copolymer werden in bevorzugten Ausführungsformen der vorliegenden Erfindung mit weiteren, oben beschriebenen Bestandteilen in kosmetischen Mitteln verwendet:

| | Silanverbindung | weiterer Inhaltsstoff |
|---|---|---|
| 1 | (3-Aminopropyl)trimethoxysilan | Acrylamidopropyltrimonium Chloride/Acrylates Copolymer |
| 2 | (3-Aminopropyl)triethoxysilan | Acrylamidopropyltrimonium Chloride/Acrylates Copolymer |
| 3 | (2-Aminoethyl)trimethoxysilan | Acrylamidopropyltrimonium Chloride/Acrylates Copolymer |
| 4 | (2-Aminoethyl)triethoxysilan | Acrylamidopropyltrimonium Chloride/Acrylates Copolymer |
| 5 | (3-Dimethylaminopropyl)trimethoxysilan | Acrylamidopropyltrimonium Chloride/Acrylates Copolymer |
| 6 | (3-Dimethylaminopropyl)triethoxysilan | Acrylamidopropyltrimonium Chloride/Acrylates Copolymer |
| 7 | (2-Dimethylaminoethyl)trimethoxysilan | Acrylamidopropyltrimonium Chloride/Acrylates Copolymer |
| 8 | (2-Dimethylaminoethyl)triethoxysilan | Acrylamidopropyltrimonium Chloride/Acrylates Copolymer |
| 9 | 3-(Trimethoxysilyl)-N-[3-(trimethoxysilyl)propyl]-1-propanamin | Acrylamidopropyltrimonium Chloride/Acrylates Copolymer |
| 10 | 3-(Triethoxysilyl)-N-[3-(triethoxysilyl)propyl]-1-propanamin | Acrylamidopropyltrimonium Chloride/Acrylates Copolymer |
| 11 | N-Methyl-3-(trimethoxysilyl)-N-[3-(trimethoxysilyl)propyl]-1-propanamin | Acrylamidopropyltrimonium Chloride/Acrylates Copolymer |
| 12 | N-Methyl-3-(triethoxysilyl)-N-[3-(triethoxysilyl)propyl]-1-propanamin | Acrylamidopropyltrimonium Chloride/Acrylates Copolymer |
| 13 | 2-[Bis[3-(trimethoxysilyl)propyl]amino]-ethanol | Acrylamidopropyltrimonium Chloride/Acrylates Copolymer |
| 14 | 2-[Bis[3-(triethoxysilyl)propyl]amino]-ethanol | Acrylamidopropyltrimonium Chloride/Acrylates Copolymer |
| 15 | 3-(Trimethoxysilyl)-N,N-bis[3-(trimethoxysilyl)propyl]-1-propanamin | Acrylamidopropyltrimonium Chloride/Acrylates Copolymer |
| 16 | 3-(Triethoxysilyl)-N,N-bis[3-(triethoxysilyl)propyl]-1-propanamin | Acrylamidopropyltrimonium Chloride/Acrylates Copolymer |
| 17 | N1,N1-Bis[3-(trimethoxysilyl)propyl]-1,2-ethandiamin | Acrylamidopropyltrimonium Chloride/Acrylates Copolymer |
| 18 | N1,N1-Bis[3-(triethoxysilyl)propyl]-1,2-ethandiamin | Acrylamidopropyltrimonium Chloride/Acrylates Copolymer |
| 19 | N,N-Bis[3-(trimethoxysilyl)propyl]-2-propen-1-amin | Acrylamidopropyltrimonium Chloride/Acrylates Copolymer |
| 20 | N,N-Bis[3-(triethoxysilyl)propyl]-2-propen-1-amin | Acrylamidopropyltrimonium Chloride/Acrylates Copolymer |
| 21 | Methyltrimethoxysilan | Acrylamidopropyltrimonium Chloride/Acrylates Copolymer |
| 22 | Methyltriethoxysilan | Acrylamidopropyltrimonium Chloride/Acrylates Copolymer |
| 23 | Ethyltrimethoxysilan | Acrylamidopropyltrimonium Chloride/Acrylates Copolymer |
| 24 | Ethyltriethoxysilan | Acrylamidopropyltrimonium Chloride/Acrylates Copolymer |
| 25 | Octyltrimethoxysilan | Acrylamidopropyltrimonium Chloride/Acrylates Copolymer |
| 26 | Octyltriethoxysilan | Acrylamidopropyltrimonium Chloride/Acrylates Copolymer |
| 27 | Dodecyltrimethoxysilan | Acrylamidopropyltrimonium Chloride/Acrylates Copolymer |
| 28 | Dodecyltriethoxysilan | Acrylamidopropyltrimonium Chloride/Acrylates Copolymer |
| 29 | Propyltrimethoxysilan | Acrylamidopropyltrimonium Chloride/Acrylates Copolymer |
| 30 | Propyltriethoxysilan | Acrylamidopropyltrimonium Chloride/Acrylates Copolymer |
| 31 | Hexyltrimethoxysilan | Acrylamidopropyltrimonium Chloride/Acrylates Copolymer |
| 32 | Hexyltriethoxysilan | Acrylamidopropyltrimonium Chloride/Acrylates Copolymer |
| 33 | Octadecyltrimethoxysilan | Acrylamidopropyltrimonium Chloride/Acrylates Copolymer |
| 34 | Octadecyltriethoxysilan | Acrylamidopropyltrimonium Chloride/Acrylates Copolymer |
| 35 | (3-Aminopropyl)triethoxysilan + Methyltrimethoxysilan | Acrylamidopropyltrimonium Chloride/Acrylates Copolymer |
| 36 | (3-Aminopropyl)triethoxysilan + Methyltriethoxysilan | Acrylamidopropyltrimonium Chloride/Acrylates Copolymer |
| 37 | (3-Aminopropyl)triethoxysilan + Ethyltrimethoxysilan | Acrylamidopropyltrimonium Chloride/Acrylates Copolymer |
| 38 | (3-Aminopropyl)triethoxysilan + Ethyltriethoxysilan | Acrylamidopropyltrimonium Chloride/Acrylates Copolymer |
| 39 | (3-Aminopropyl)triethoxysilan + Propyltrimethoxysilan | Acrylamidopropyltrimonium Chloride/Acrylates Copolymer |
| 40 | (3-Aminopropyl)triethoxysilan + Propyltriethoxysilan | Acrylamidopropyltrimonium Chloride/Acrylates Copolymer |
| 41 | (3-Aminopropyl)triethoxysilan + Hexyltrimethoxysilan | Acrylamidopropyltrimonium Chloride/Acrylates Copolymer |
| 42 | (3-Aminopropyl)triethoxysilan + Hexyltriethoxysilan | Acrylamidopropyltrimonium Chloride/Acrylates Copolymer |
| 43 | (3-Aminopropyl)triethoxysilan + Octyltrimethoxysilan | Acrylamidopropyltrimonium Chloride/Acrylates Copolymer |
| 44 | (3-Aminopropyl)triethoxysilan + Octyltriethoxysilan | Acrylamidopropyltrimonium Chloride/Acrylates Copolymer |
| 45 | (3-Aminopropyl)triethoxysilan + Dodecyltrimethoxysilan | Acrylamidopropyltrimonium Chloride/Acrylates Copolymer |
| 46 | (3-Aminopropyl)triethoxysilan + Dodecyltriethoxysilan | Acrylamidopropyltrimonium Chloride/Acrylates Copolymer |
| 47 | (3-Aminopropyl)triethoxysilan + Octadecyltrimethoxysilan | Acrylamidopropyltrimonium Chloride/Acrylates Copolymer |
| 48 | (3-Aminopropyl)triethoxysilan + Octyldecyltriethoxysilan | Acrylamidopropyltrimonium Chloride/Acrylates Copolymer |

Die Wirkstoffkombination der organischen Siliciumverbindung und dem Polyacrylat-Copolymer kann bereits im Mittel zur Behandlung eines keratinischen Materials enthalten sein. In dieser Ausführungsform wird das Mittel zur Behandlung eines keratinischen Materials bereits in anwendungsbereiter Form vertrieben. Um eine möglichst lagerstabile Formulierung bereitstellen zu können, wird das Mittel selbst bevorzugt wasserarm oder wasserfrei konfektioniert.

Alternativ wird die mindestens eine organische Siliciumverbindung maximal 12 Stunden, bevorzugt maximal 6 Stunden, mehr bevorzugt maximal 3 Stunde, noch mehr bevorzugt maximal 1 Stunde vor Anwendung des Mittels zur Behandlung eines keratinischen Materials einer Basis umfassend alle Inhaltsstoffe des Mittels zur Behandlung eines keratinischen Materials mit Ausnahme der mindestens einen organischen Siliciumverbindung zugefügt.

Ferner wird alternativ die organische Siliciumverbindung einem kosmetischen Produkt erst kurz vor der Anwendung, d.h. 1 Minute bis 12 Stunden, bevorzugt 2 Minuten bis 6 Stunden, besonders bevorzugt 1 Minute bis 3 Stunden, insbesondere bevorzugt 1 Minute bis 1 Stunde, zugegeben.

In einer weiteren Alternative wird die organische Siliciumverbindung einer wässrigen Lösung zugegeben, welche auf das Haar appliziert wird und im zweiten Schritt wird eine wässrige Lösung oder ein kosmetisches Mittel, welches das Polyacrylat-Copolymerenthält, auf das Haar aufgetragen.

Beispielsweise kann der Anwender ein Mittel (α), welches die organische Siliciumverbindung(en) enthält, zunächst mit einem Mittel (β), welches die restlichen Inhaltsstoffe des Mittels zur Behandlung eines keratinischen Materials umfasst, verrühren oder verschütteln. Diese Mischung aus (α) und (β) kann der Anwender nun - entweder direkt nach ihrer Herstellung oder nach einer kurzen Reaktionszeit von 1 Minute bis 20 Minuten - auf die keratinischen Materialien applizieren. Das Mittel (β) kann Wasser enthalten, insbesondere Wasser in einer Menge > 30 Gew.-%, bezogen auf das Gesamtgewicht des Mittels zur Behandlung keratinischer Materialien.

Ein weiterer Gegenstand der vorliegenden Anmeldung ist die Verwendung eines erfindungsgemäßen kosmetischen Mittels zur Behandlung eines keratinischen Materials zum Auswaschschutz zum Auswaschschutz von gefärbtem keratinischen Material, zur Formgebung von keratinischem Material und/oder zur Pflege von keratinischem Material.

Bezüglich weiterer bevorzugter Ausführungsformen der Verwendung gilt mutatis mutandis das zu den kosmetischen Mitteln Gesagte.

## Patentansprüche

1. Kosmetisches Mittel zur Behandlung eines keratinischen Materials, umfassend
a) mindestens eine organische Siliciumverbindung, wobei die mindestens eine organische Siliciumverbindung eine Verbindung der Formel (I) und mindestens eine organische Siliciumverbindung der Formel (IV) enthält,
wobei in der organischen Siliciumverbindung der Formel (I)
R₁R₂N-L-Si(OR₃)ₐ(R₄)_{b} (I),
- R₁, R₂ beide für ein Wasserstoffatom stehen,
- L für eine lineare, zweibindige C₁-C₆-Alkylengruppe, bevorzugt für eine Propylengruppe (-CH₂-CH₂-CH₂-) oder für eine Ethylengruppe (-CH₂-CH₂-), steht,
- R₃, R₄ unabhängig voneinander für eine Methylgruppe oder für eine Ethylgruppe stehen,
- a für die Zahl 3 steht und
- b für die Zahl 0 steht, und
wobei in der organischen Siliciumverbindung der Formel (IV)
R₉Si(OR₁₀)ₖ(R₁₁)ₘ (IV),
- R₉ für eine C₁-C₁₂-Alkylgruppe steht,
- R₁₀ für ein Wasserstoffatom oder eine C₁-C₆-Alkylgruppe steht,
- R₁₁ für eine C₁-C₆-Alkylgruppe steht
- k für eine ganze Zahl von 1 bis 3 steht, und
- m für die ganze Zahl 3 - k steht,die ein bis drei Siliciumatome enthält, und
b) mindestens ein Polyacrylat-Copolymer, das gebildet wird aus den Monomeren Acrylsäure und Acrylamidopropyltrimoniumchlorid.

2. Kosmetisches Mittel zur Behandlung eines keratinischen Materials gemäß Anspruch 1, **dadurch gekennzeichnet,**
**dass** die mindestens eine organische Siliciumverbindung ferner eine Verbindung der Formel (II) enthält,
wobei in der organischen Siliciumverbindung der Formel (II)
(R₅O)_{c}(Rₑ)_{d}Si-(A)ₑ-[NR₇-(A')]_{f}-[O-(A")]_{g}-[NRₑ-(A‴)]ₕ-Si(R₆')_{d'}(OR₅')_{c'} (II),
- R₅, R_{5'}, R_{5"}, R₆, R_{6'} und R_{6"} unabhängig voneinander für eine C₁-C₆-Alkylgruppe stehen,
- A, A', A", A‴ und A"" unabhängig voneinander für eine lineare oder verzweigte, zweibindige C₁-C₂₀-Alkylengruppe stehen,
- R₇ und R₈ unabhängig voneinander für ein Wasserstoffatom, eine C₁-C₆-Alkylgruppe, eine Hydroxy-C₁-C₆-alkylgruppe, eine C₂-C₆-Alkenylgruppe, eine Amino-C₁-C₆-Alkylgruppe oder eine Gruppierung der Formel (III) stehen
- (Aʺʺ)-Si(R₆'')_{d"}(OR₅'')_{c"} (III),
- c für eine ganze Zahl von 1 bis 3 steht,
- d für die ganze Zahl 3 - c steht,
- c' für eine ganze Zahl von 1 bis 3 steht,
- d' für die ganze Zahl 3 - c' steht,
- c" für eine ganze Zahl von 1 bis 3 steht,
- d" für die ganze Zahl 3 - c" steht,
- e für 0 oder 1 steht,
- f für 0 oder 1 steht,
- g für 0 oder 1 steht,
- h für 0 oder 1 steht,
mit der Maßgabe, dass mindestens einer der Reste aus e, f, g und h von 0 verschieden ist.

3. Kosmetisches Mittel zur Behandlung eines keratinischen Materials gemäß einem der Ansprüche 1 oder 2,
**dadurch gekennzeichnet,**
**dass** das Mittel zur Behandlung eines keratinischen Materials mindestens eine organische Siliciumverbindung der Formel (I) enthält, die ausgewählt ist aus der Gruppe bestehend aus
- (3-Aminopropyl)trimethoxysilan
- (3-Aminopropyl)triethoxysilan
- (2-Aminoethyl)trimethoxysilan und
- (2-Aminoethyl)triethoxysilan.

4. Kosmetisches Mittel zur Behandlung eines keratinischen Materials gemäß einem der Ansprüche 1 bis 3, **dadurch gekennzeichnet, dass** die organische Siliciumverbindung der Formel (I) in einer Menge von 0,01 bis 10 Gew.-%, bevorzugt von 0,02 bis 8 Gew.-%, bevorzugter von 0,05 bis 6 Gew.-%, am meisten bevorzugt von 0,1 bis 4 Gew.-%, bezogen auf das Gesamtgewicht des kosmetischen Mittels, in dem kosmetischen Mittel enthalten ist.

5. Kosmetisches Mittel zur Behandlung eines keratinischen Materials gemäß einem der Ansprüche 1 bis 4, **dadurch gekennzeichnet, dass** das Mittel zur Behandlung eines keratinischen Materials mindestens eine organische Siliciumverbindung der Formel (IV) enthält,
R₉Si(OR₁₀)ₖ(R₁₁)ₘ (IV),
die ausgewählt ist aus der Gruppe bestehend aus
- Methyltrimethoxysilan
- Methyltriethoxysilan
- Ethyltrimethoxysilan
- Ethyltriethoxysilan
- Propyltrimethoxysilan
- Propyltriethoxysilan
- Hexyltrimethoxysilan
- Hexyltriethoxysilan
- Octyltrimethoxysilan
- Octyltriethoxysilan
- Dodecyltrimethoxysilan und
- Dodecyltriethoxysilan.

6. Kosmetisches Mittel zur Behandlung eines keratinischen Materials gemäß einem der Ansprüche 1 bis 5, **dadurch gekennzeichnet, dass** das Polyacrylat-Copolymer aus den folgenden Monomeren: 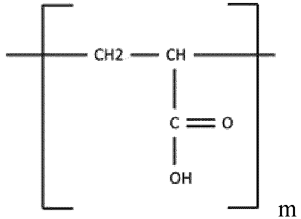 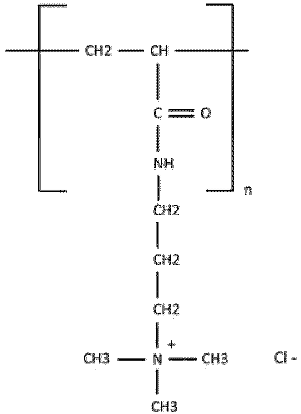 gebildet wird, wobei n und m die Anzahl der Monomereinheiten darstellt, wobei das Verhältnis von m zu n zwischen 90 zu 10 bis 10 zu 90, bevorzugt zwischen 75 zu 25 bis 25 zu 75, bevorzugter zwischen 60 zu 40 und 40 zu 60, liegt.

7. Kosmetisches Mittel zur Behandlung eines keratinischen Materials gemäß einem der Ansprüche 1 bis 6, **dadurch gekennzeichnet, dass** das Polyacrylat-Copolymer in einer Menge von 0,01 bis 10 Gew.-%, bevorzugt von 0,25 bis 8 Gew.-%, bevorzugter von 0,5 bis 6 Gew.-%, jeweils bezogen auf das Gesamtgewicht des kosmetischen Mittels, in dem kosmetischen Mittel enthalten ist.

8. Kosmetisches Mittel zur Behandlung eines keratinischen Materials gemäß einem der Ansprüche 1 bis 7, **dadurch gekennzeichnet, dass** das kosmetische Mittel ferner eine Komponente c) enthält, die bevorzugt ausgewählt ist aus der Gruppe bestehend aus Glycerin, Harnstoff, Hyaluronsäure, Silanolester der Hyaluronsäure, Panthenol, Taurin, Ceramiden, Phytosterolen, Aloe Vera Extrakten, Kreatin, Kreatinin, Natriumhyaluronat, Polysacchariden, Biosaccharide gum-1, Gurkenextrakten, Butylenglykol, Propylenglykol, Methylpropandiol, Ethylhexylglycerin, Sorbitol, Aminosäuren, wobei Glycin, Glycin Soja, Histidin, Tyrosin oder Tryptophan besonders bevorzugte Aminosäuren sind, Aminosäurederivaten, Milchsäure, Lactaten, insbesondere Natriumlactat, und/oder Ethylhexyloxyglycerin.

9. Kosmetisches Mittel zur Behandlung eines keratinischen Materials gemäß einem der Ansprüche 1 bis 8, **dadurch gekennzeichnet, dass** das Mittel zur Behandlung eines keratinischen Materials- bezogen auf das Gesamtgewicht des Mittels zur Behandlung eines keratinischen Materials - enthält:
- 0,5 bis 3 Gew.-% mindestens einer ersten organischen Siliciumverbindung, die ausgewählt ist aus der Gruppe aus (3-Aminopropyl)trimethoxysilan, (3-Aminopropyl)triethoxysilan, (2-Aminoethyl)trimethoxysilan und (2-Aminoethyl)triethoxysilan, und
- 3,2 bis 7 Gew.-% mindestens einer zweiten organischen Siliciumverbindung, die ausgewählt ist aus der Gruppe aus Methyltrimethoxysilan, Methyltriethoxysilan, Ethyltrimethoxysilan, Ethyltriethoxysilan, Propyltrimethoxysilan, Propyltriethoxysilan, Hexyltrimethoxysilan, Hexyltriethoxysilan, Octyltrimethoxysilan, Octyltriethoxysilan, Dodecyltrimethoxysilan, Dodecyltriethoxysilan, Octadecyltrimethoxysilan und Octadecyltriethoxysilan.

10. Verwendung eines kosmetischen Mittels zur Behandlung eines keratinischen Materials gemäß einem der Ansprüche 1 bis 9
zum Auswaschschutz von gefärbtem keratinischen Material,
zur Formgebung von keratinischem Material und/oder
zur Pflege von keratinischem Material.

## Claims

1. A cosmetic agent for treating a keratinous material, the agent comprising
a) at least one organosilicon compound, wherein the at least one organosilicon compound contains a compound of formula (I) and at least one organosilicon compound of formula (IV),
where, in the organosilicon compound of formula (I),
R₁R₂N-L-Si(OR₃)ₐ(R₄)_{b} (I),
- R₁ and R₂ both represent a hydrogen atom,
- L represents a linear, divalent C₁-C₆ alkylene group, preferably a propylene group (-CH₂-CH₂-CH₂-) or an ethylene group (-CH₂-CH₂-),
- R₃ and R₄ independently represent a methyl group or an ethyl group,
- a represents the number 3, and
- b represents the number 0, and
where, in the organosilicon compound of formula (IV),
R₉Si(OR₁₀)ₖ(R₁₁)ₘ (IV),
- R₉ represents a C₁-C₁₂-alkyl group,
- R₁₀ represents a hydrogen atom or a C₁-C₆ alkyl group,
- R₁₁ represents a C₁-C₆ alkyl group,
- k represents an integer from 1 to 3, and
- m represents the integer 3 - k, which contains one to three silicon atoms, and
b) at least one polyacrylate copolymer formed by the monomers acrylic acid and acrylamidopropyltrimonium chloride.

2. The cosmetic agent for treating a keratinous material according to claim 1, **characterized in that**
the at least one organosilicon compound further contains a compound of formula (II),
where, in the organosilicon compound of formula (II),
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R₅, R_{5'}, R_{5"}, R₆, R_{6'} and R_{6"} independently represent a C₁-C₆ alkyl group,
- A, A', A", A‴ and Aʺʺ independently represent a linear or branched, divalent C₁-C₂₀ alkylene group,
- R₇ and R₈ independently represent a hydrogen atom, a C₁-C₆-alkyl group, a hydroxy C₁-C₆-alkyl group, a C₂-C₆-alkenyl group, an amino C₁-C₆-alkyl group, or a grouping of formula (III),
- (Aʺʺ)-Si(R₆")_{d"}(OR₅")_{c"} (III),
- c represents an integer from 1 to 3,
- d represents the integer 3 - c,
- c' represents an integer from 1 to 3,
- d' represents the integer 3 - c',
- c" represents an integer from 1 to 3,
- d" represents the integer 3 - c",
- e represents 0 or 1,
- f represents 0 or 1,
- g represents 0 or 1,
- h represents 0 or 1,
with the proviso that at least one of the functional groups e, f, g and h is different from 0.

3. The cosmetic agent for treating a keratinous material according to one of claims 1 or 2,
**characterized in that**
the agent for treating a keratinous material contains at least one organosilicon compound of formula (I), which is selected from the group consisting of
- (3-aminopropyl)trimethoxysilane,
- (3-aminopropyl)triethoxysilane,
- (2-aminoethyl)trimethoxysilane, and
- (2-aminoethyl)triethoxysilane.

4. The cosmetic agent for treating a keratinous material according to one of claims 1 to 3, **characterized in that** the organosilicon compound of formula (I) is contained in the cosmetic agent in an amount of from 0.01 to 10 wt.%, preferably from 0.02 to 8 wt.%, more preferably from 0.05 to 6 wt.%, most preferably from 0.1 to 4 wt.%, based on the total weight of the cosmetic agent.

5. The cosmetic agent for treating a keratinous material according to one of claims 1 to 4, **characterized in that** the agent for treating a keratinous material contains at least one organosilicon compound of formula (IV),
R₉Si(OR₁₀)ₖ(R₁₁)ₘ (IV),
which is selected from the group consisting of
- methyltrimethoxysilane,
- methyltriethoxysilane,
- ethyltrimethoxysilane,
- ethyltriethoxysilane,
- propyltrimethoxysilane,
- propyltriethoxysilane,
- hexyltrimethoxysilane,
- hexyltriethoxysilane,
- octyltrimethoxysilane,
- octyltriethoxysilane,
- dodecyltrimethoxysilane, and
- dodecyltriethoxysilane.

6. The cosmetic agent for treating a keratinous material according to one of claims 1 to 5, **characterized in that** the polyacrylate copolymer is formed by the following monomers: 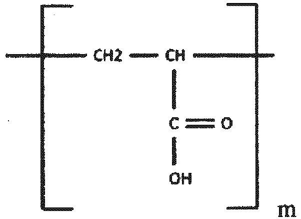 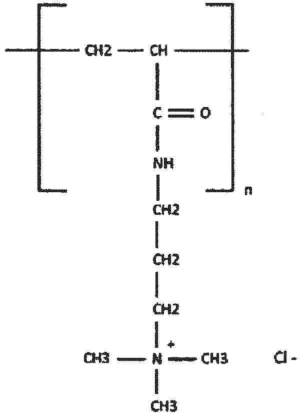 where n and m represent the number of monomer units, where the ratio of m to n is between 90 to 10 to 10 to 90, preferably between 75 to 25 to 25 to 75, more preferably between 60 to 40 and 40 to 60.

7. The cosmetic agent for treating a keratinous material according to one of claims 1 to 6, **characterized in that** the polyacrylate copolymer is contained in the cosmetic agent in an amount of from 0.01 to 10 wt.%, preferably from 0.25 to 8 wt.%, more preferably from 0.5 to 6 wt.%, in each case based on the total weight of the cosmetic agent.

8. The cosmetic agent for treating a keratinous material according to one of claims 1 to 7, **characterized in that** the cosmetic agent further contains a component c) which is preferably selected from the group consisting of glycerol, urea, hyaluronic acid, silanol esters of hyaluronic acid, panthenol, taurine, ceramides, phytosterols, aloe vera extracts, creatine, creatinine, sodium hyaluronate, polysaccharides, biosaccharide gum-1, cucumber extracts, butylene glycol, propylene glycol, methylpropanediol, ethylhexylglycerol, sorbitol, amino acids, wherein glycine, glycine soja, histidine, tyrosine or tryptophan are particularly preferred amino acids, amino acid derivatives, lactic acid, lactates, in particular sodium lactate, and/or ethylhexyloxyglycerol.

9. The cosmetic agent for treating a keratinous material according to one of claims 1 to 8, **characterized in that** the agent for treating a keratinous material contains, based on the total weight of the agent for treating a keratinous material:
- 0.5 to 3 wt.% of at least one first organosilicon compound selected from the group consisting of (3-aminopropyl)trimethoxysilane, (3-aminopropyl)triethoxysilane, (2-aminoethyl)trimethoxysilane and (2-aminoethyl)triethoxysilane, and
- 3.2 to 7 wt.% of at least one second organosilicon compound selected from the group consisting of methyltrimethoxysilane, methyltriethoxysilane, ethyltrimethoxysilane, ethyltriethoxysilane, propyltrimethoxysilane, propyltriethoxysilane, hexyltrimethoxysilane, hexyltriethoxysilane, octyltrimethoxysilane, octyltriethoxysilane, dodecyltrimethoxysilane, dodecyltriethoxysilane, octadecyltrimethoxysilane and octadecyltriethoxysilane.

10. The use of a cosmetic agent for treating a keratinous material according to one of claims 1 to 9
for washout protection of colored keratinous material,
for shaping keratinous material and/or
for maintaining keratinous material.

## Revendications

1. Agent cosmétique destiné à traiter une matière kératinique, comprenant
a) au moins un composé organique de silicium, dans lequel l'au moins un composé organique de silicium contient un composé de formule (I) et au moins un composé organique de silicium de formule (IV),
où, dans le composé organique de silicium de formule (I)
R₁R₂N-L-Si(OR₃)ₐ(R₄)_{b} (I),
- R₁, R₂ représentent tous deux un atome d'hydrogène,
- L représente un groupe alkylène en C₁-C₆ bivalent linéaire, de préférence un groupe propylène (-CH₂-CH₂-CH₂-) ou un groupe éthylène (-CH₂-CH₂),
- R₃, R₄ représentent, indépendamment l'un de l'autre, un groupe méthyle ou un groupe éthyle,
- a représente le nombre 3 et
- b représente le nombre 0, et
où, dans le composé organique de silicium de formule (IV)
R₉Si(OR₁₀)ₖ(R₁₁)ₘ (IV),
- R₉ représente un groupe alkyle en C₁-C₁₂,
- R₁₀ représente un atome d'hydrogène ou un groupe alkyle en C₁-C₆,
- R₁₁ représente un groupe alkyle en C₁-C₆,
- k représente un nombre entier allant de 1 à 3, et
- m représente le nombre entier 3 - k qui contient un à trois atomes de silicium, et
b) au moins un copolymère de polyacrylate qui est formé à partir des monomères d'acide acrylique et de chlorure d'acrylamidopropyltrimonium.

2. Agent cosmétique destiné à traiter une matière kératinique selon la revendication 1, **caractérisé en ce**
**que** l'au moins un composé organique de silicium contient en outre un composé de formule (II),
où, dans le composé organique de silicium de formule (II)
(R₅O)_{c}(R₆)_{d}Si-(A)ₑ-[NR₇-(A')]_{f}-[O-(A")]_{g}-[NR₈-(A‴)]ₕ-Si(R₆')_{d'}(OR₅')_{c'} (II),
- R₅, R_{5'}, R_{5"}, R₆, R_{6'} et R_{6"} représentent, indépendamment les uns des autres, un groupe alkyle en C₁-C₆,
- A, A', A", A‴ et Aʺʺ représentent, indépendamment les uns des autres, un groupe alkylène en C₁-C₂₀ bivalent, linéaire ou ramifié,
- R₇ et R₈ représentent, indépendamment l'un de l'autre, un atome d'hydrogène, un groupe alkyle en C₁-C₆, un groupe hydroxy alkyle en C₁-C₆, un groupe alcényle en C₂-C₆, un groupe amino alkyle en C₁-C₆ ou un groupement de formule (III)
- (Aʺʺ)-Si(R₆")_{d"}(OR₅")_{c"} (III),
- c représente un nombre entier allant de 1 à 3,
- d représente le nombre entier 3 - c,
- c' représente un nombre entier allant de 1 à 3,
- d' représente le nombre entier 3 - c',
- c" représente un nombre entier allant de 1 à 3,
- d" représente le nombre entier 3 - c",
- e représente 0 ou 1,
- f représente 0 ou 1,
- g représente 0 ou 1,
- h représente 0 ou 1,
à condition qu'au moins l'un des radicaux parmi e, f, g et h soit différent de 0.

3. Agent cosmétique destiné à traiter une matière kératinique selon l'une des revendications 1 ou 2,
**caractérisé en ce**
**que** l'agent destiné à traiter une matière kératinique contient au moins un composé organique de silicium de formule (I) qui est choisi dans le groupe constitué de
- (3-aminopropyl)triméthoxysilane
- (3-aminopropyl)triéthoxysilane
- (2-aminoéthyl)triméthoxysilane et
- (2-aminoéthyl)triéthoxysilane.

4. Agent cosmétique destiné à traiter une matière kératinique selon l'une des revendications 1 à 3, **caractérisé en ce que** le composé organique de silicium de formule (I) est contenu dans l'agent cosmétique en une quantité allant de 0,01 à 10 % en poids, de préférence de 0,02 à 8 % en poids, plus préférablement de 0,05 à 6 % en poids, le plus préférablement de 0,1 à 4 % en poids, par rapport au poids total de l'agent cosmétique.

5. Agent cosmétique destiné à traiter une matière kératinique selon l'une des revendications 1 à 4, **caractérisé en ce que** l'agent destiné à traiter une matière kératinique contient au moins un composé organique de silicium de formule (IV)
R₉Si(OR₁₀)ₖ(R₁₁)ₘ (IV),
lequel est choisi dans le groupe constitué de
- méthyltriméthoxysilane
- méthyltriéthoxysilane
- éthyltriméthoxysilane
- éthyltriéthoxysilane
- propyltriméthoxysilane
- propyltriéthoxysilane
- hexyltriméthoxysilane
- hexyltriéthoxysilane
- octyltriméthoxysilane
- octyltriéthoxysilane
- dodécyltriméthoxysilane et
- dodécyltriéthoxysilane.

6. Agent cosmétique destiné à traiter une matière kératinique selon l'une des revendications 1 à 5, **caractérisé en ce que** le copolymère de polyacrylate est formé à partir des monomères suivants : 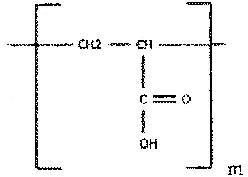 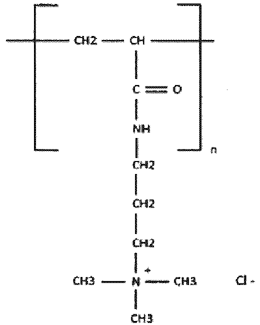 où n et m représentent le nombre de motifs monomères, dans lequel le rapport de m à n est compris entre 90 à 10 à 10 à 90, de préférence entre 75 à 25 à 25 à 75, plus préférablement entre 60 à 40 et 40 à 60.

7. Agent cosmétique destiné à traiter une matière kératinique selon l'une des revendications 1 à 6, **caractérisé en ce que** le copolymère de polyacrylate est contenu dans l'agent cosmétique en une quantité allant de 0,01 à 10 % en poids, de préférence de 0,25 à 8 % en poids, plus préférablement de 0,5 à 6 % en poids, respectivement par rapport au poids total de l'agent cosmétique.

8. Agent cosmétique destiné à traiter une matière kératinique selon l'une des revendications 1 à 7, **caractérisé en ce que** l'agent cosmétique contient en outre un composant c), lequel est de préférence choisi dans le groupe constitué de glycérine, urée, acide hyaluronique, ester de silanol de l'acide hyaluronique, panthénol, taurine, céramides, phytostérols, extraits d'Aloe vera, créatine, créatinine, hyaluronate de sodium, polysaccharides, biosaccharide gum-1, extraits de concombre, butylène glycol, propylène glycol, méthylpropanediol, éthylhexylglycérine, sorbitol, acides aminés, dans lequel la glycine, la glycine de soja, l'histidine, la tyrosine ou le tryptophane sont des acides aminés particulièrement préférés, dérivés d'acides aminés, acide lactique, lactates, en particulier lactate de sodium, et/ou éthylhexyloxyglycérine.

9. Agent cosmétique destiné à traiter une matière kératinique selon l'une des revendications 1 à 8, **caractérisé en ce que** l'agent destiné à traiter une matière kératinique contient, par rapport au poids total de l'agent destiné à traiter une matière kératinique :
- 0,5 à 3 % en poids d'au moins un premier composé organique de silicium qui est choisi dans le groupe constitué de (3-aminopropyl)triméthoxysilane, (3-aminopropyl)triéthoxysilane, (2-aminoéthyl)triméthoxysilane et (2-aminoéthyl)triéthoxysilane, et
- 3,2 à 7 % en poids d'au moins un second composé organique de silicium qui est choisi dans le groupe constitué de méthyltriméthoxysilane, méthyltriéthoxysilane, éthyltriméthoxysilane, éthyltriéthoxysilane, propyltriméthoxysilane, propyltriéthoxysilane, hexyltriméthoxysilane, hexyltriéthoxysilane, octyltriméthoxysilane, octyltriéthoxysilane, dodécyltriméthoxysilane, dodécyltriéthoxysilane, octadécyltriméthoxysilane et octadécyltriéthoxysilane.

10. Utilisation d'un agent cosmétique destiné à traiter une matière kératinique selon l'une des revendications 1 à 9
pour la protection contre le délavage de la matière kératinique colorée,
pour la mise en forme de la matière kératinique et/ou
pour le soin de la matière kératinique.
